Europäisches Patentamt

European Patent Office

Office européen des brevets

(19)

(11) Publication number : **0 465 235 A1**

# (12) EUROPEAN PATENT APPLICATION

(21) Application number : **91306030.7**

(22) Date of filing : **03.07.91**

(51) Int. Cl.$^5$ : **A61K 45/06, A61K 33/00**

(30) Priority : **03.07.90 US 547663**

(43) Date of publication of application :
**08.01.92 Bulletin 92/02**

(84) Designated Contracting States :
**DE ES GB IT**

(71) Applicant : **McNEIL-PPC, Inc.**
**Van Liew Avenue**
**Milltown New Jersey 08850 (US)**

(72) Inventor : **Goldman, William J.**
**855 Lewis Lane**
**Ambler, Pennsylvania 19002 (US)**

(74) Representative : **Mercer, Christopher Paul et al**
**Carpmaels & Ransford 43, Bloomsbury Square**
**London WC1A 2RA (GB)**

(54) **Pharmaceutical compositions and methods for alleviating gastrointestinal symptoms induced by nonsteroidal anti-inflammatory drugs.**

(57) This invention relates to an analgesic pharmaceutical composition for treating pain and the symptoms of nonsteroidal anti-inflammatory drug induced gastric acidity comprising an analgesic effective amount of a nonsteroidal anti-inflammatory drug and a gastric acid neutralizing effective amount of an antacid and methods of treating the symptoms of nonsteroidal anti-inflammatory drug induced gastric acidity comprising administering such pharmaceutical compositions.

EP 0 465 235 A1

## Field of the Invention

This invention relates to novel pharmaceutical compositions for preventing or treating gastrointestinal symptoms induced by nonsteroidal anti-inflammatory drugs. More particularly, the invention comprises treating the gastrointestinal symptoms induced by nonsteroidal anti-inflammatory drugs with a combination of a nonsteroidal anti-inflammatory drug with an antacid buffering agent composition.

## Background of the invention

Nonsteroidal anti-inflammatory drugs (hereinafter referred to as "NSAID(s)") are known to be effective analgesics and anti-inflammatory agents. Buffering agents are alkaline materials which may be used to reduce the acidity of various compositions and solutions including drugs. The presence of alkaline material has been shown to alter gastrointestinal irritancy of a number of NSAIDS (See Bollet, A.J., "Nonsteroidal Anti-inflammatory Drugs." Textbook of Rheumatology, 2nd edition, W. B. Saunders Co., pp. 761-762, 1985).

While aspirin is a commonly used NSAID for relief of minor aches and pains, nonaspirin NSAIDs provide clinically superior analgesic, anti-inflammatory and antipyretic activity and are particularly wall suited for treatment of acute and chronic pain associated with arthritis and other muscular and skeletal disorders. This invention focuses on the therapeutic use of nonaspirin NSAID(s) and providing novel NSAID combinations.

The occurence of gastrointestinal discomfort associated with the use of nonsteroidal anti-inflammatory drugs is well known (See Pemberton, R.E. and L.J. Strand, "A Review of Upper-Gastrointestinal Effects of the Newer Nonsteroidal Anti-inflammatory Agents", Digestive Diseases and Sciences, 24:53-64, 1979; Silvoso, G.R., K.J. Ivey, J.H. Butt, "Incidence of Gastric Lesions in Patients with Rheumatic Disease on Chronic Aspirin Therapy", Am. Intern. Med.: 91, 517-520, 1979). Ehsanullah and co-workers (See Ehsanullah, R.S.B., M.C. page, G. Tildesley and J.R. Wood. "Prevention of gastroduodenal damage induced by nonsteroidal anti-inflammatory drugs: controlled trial of ranitidine", BMJ 297:1017-1021, 1988) have indicated that up to 60% of patients on chronic NSAID therapy report dyspepsia as a side effect of treatment. The main toxicity of the NSAIDs occurs in the gastrointestinal tract (See Bollet, A.J., "Nonsteroidal anti-inflammatory Drugs", Textbook of Rheumatology, 2nd Edition, W.B. Saunders Company, p. 761, 1985). Epigastric distress, nausea, vomiting and occasionally more serious side effects such as upper gastrointestinal bleeding are undesirable effects common to almost all studies relating to these drugs.

It is believed that more than 15 million Americans take NSAIDs (See Roth, S.H., NSAID and Gastropathy: A Rheumatologists's Review. J. Rheum. 15: 912-919, 1988). Forty-four percent of all physical disability which occurs in older patients are related to the aches and pains associated with arthritic disorders (See Wilson, D.E. Use of Misoprostol to prevent NSAID-Associated Gastric Ulcers: Geriatric Med. Today 8:82-88, 1989). Due to the development of asymptomatic gastric ulcerations, the relationship between symptoms and mucosal damage have not been able to be fully demonstrated. (See Marble, D.A., and J.R. Ward, Managing NSAID-Induced Peptic Ulcers: Drug Therapy, pp. 34-46; Sep., 1989). These references suggest that the reason for the development of silent ulceration is the fact that the NSAIDs are effective analgesics and hence are capable of masking the symptoms of gastric irritation.

Endoscopic evaluations of patients taking NSAIDs chronically have found a 20% incidence in the rate of gastrointestinal ulcerations (See Guth, P.H., The Ulcer Patient and Nonsteroidal Anti-inflammatory Drugs Pract. Gastroent. Special Supplement, p. 12-15, May/June 1988). Other studies have shown that most if not all other NSAIDs are implicated in causing gastric and duodenal mucosal damage even if taken on a short-term basis and even more so in those patients taking NSAIDs chronically. (See Larkai, E.N. et al., Amer. J. Gastroenterol. 82:1153, 1987), including aspirin, ibuprofen, indomethacin, phenylbutazone, fenoprofen, tolmetin, naproxen and ketoprofen.

While discontinuation of the particular offending NSAID would eliminate this problem for many patients, NSAID therapy represents a primary medication in clinical situations where alternative therapy is unacceptable. Wilson (Wilson, D.E., Use of Misoprostol to Prevent NSAID-Associated Gastric Ulcers. Geriatric Med. Today 8:82-88, 1989) has stated that NSAIDs can damage gastrointestinal mucosa by absorption into the gastric mucosal cells and disrupting the gastric mucosal barrier permitting acid to diffuse into the gastric mucosa and cause damage thereto. While the NSAIDs anti-inflammatory activity is beneficial for a multitude of patients it is precisely this activity which is associated with inducing gastric mucosal damage.

B.S. Bloom in Risk and Cost of Gastrointestinal Side Effects Associated with Nonsteroidal Anti-inflammatory Drugs: Arch Intern. Med. 149:1019-1022, 1989 notes that alternative concomitant therapy is frequently necessary in NSAID treated patients to alleviate the adverse gastrointestinal side effects which occur. This concomitant therapy has previously included the separate administration of antacids, $H_2$ blockers, gastric mucosal protectants and more recently prostaglandin analogues. Harvey, (See Harvey, S.C. "Gastric Antacids and Digestants," The Pharmacological Basis of Therapeutics, 7th edition, MacMillan Publishing Co., N.Y. p. 960-975, 1985) has noted that antacids have an ability to neutralize or remove acid from the gastric contents and have been used to treat hyper chlorhydria, peptic ulcer and a variety of gastric symptoms including heartburn and sour stomach.

Previous studies (See VA Gastric Ulcer Study Group. The Veterans Administration cooperative study on gastric ulcer. Gastroenterology 1:881-883, 1971) have shown that treatment with antacids results in a 76% healing rate in patients with gastric ulcers. Not only has it been demonstrated that antacids will result in healing of gastric ulcers as noted above, but also in many of these patients appropriate levels of antacids will relieve the pain associated with the peptic ulcer. Harvey states that in order to relieve the pain induced by mucosal damage, it is only necessary to raise the gastric pH from a normal level of 1.3 to a level of 2.0. This alkalinizatin is due to an interaction between the antacid and the acid contents of the stomach.

The treatment of the aches and pains associated with arthritic disorders or muscular or skeletal disorders, e.g. bursitis or tendinitis, requires the administration of an NSAID. Reduction or treatment of the symptoms of NSAID induced gastric irritancy can be accomplished by the co-administration of an antacid along with the NSAID or the administration of NSAID with food or immediately after meals. Except for aspirin/alkaline combinations, no NSAID/antacid products are commercially available.

The suggested separate co-administration of NSAIDs with food or antacids provides obvious practical problems. Adjusting dosage regimens to meal schedules is impractical in a daily routine for patients and may present interaction problems with food and the NSAID which may effect the bioavailability, efficacy or analgesic onset speed of the NSAID. Further, dual administration of separate medications simultaneously presents problems of patient compliance and professional administration particularly, in hospitals and nursing homes.

The concept of combining a non-aspirin NSAID with an agent to lower the gastric acidity of NSAID, reduce acid production associated with its administration and provide a higher alkaline level in order to prevent mucosal damage due to NSAID therapy in a single composition which overcomes the problems of dual administration with either meals or antacids has heretofore been unrecognized. Such a combination which can be tailored to provide optional amounts of antacids to efficiently and economically buffer NSAIDs is a significant advance and meets a long felt need for providing a single composition to effectively treat arthritic disorders with an NSAID while mitigating or eliminating NSAID-induced acidity.

Summary of the Invention

The foregoing object of fulfilling a long felt need for pharmaceutical compositions which can relieve the symptoms of pain, e.g. arthritis by means of NSAIDs while alleviating NSAID induced gastric acidity has now been accomplished in accordance with the compositions and methods of the present invention.

In accordance with the purposes of the invention, as embodied and fully described herein, the invention comprises analgesic pharmaceutical compositions for treating humans suffering from pain and alleviating the gastrointestinal symptoms induced by the administration of NSAIDs comprising a combination of an analgesic anti-inflammatory effective amount of a nonaspirin NSAID and a gastric acid neutralizing effective amount of an antacid.

In preferred embodiments the NSAID is selected from the group consisting of propionic acid derivatives including ibuprofen, naproxen, fenoprofen, flurbiprofen and ketoprofen; fenamic acid derivatives, including meclofenamate and mefenamic acid; oxicams, including piroxicam; indole acetic acids, including indomethacin, sulindac, tolmetin; and pharmaceutically acceptable salts thereof. The preferred antacids are selected from the group of antacids including aluminum hydroxide, basic aluminum carbonate, dihydroxyaluminum sodium carbonate, dihydroxyaluminum aminoacetate, calcium carbonate, magnesium carbonate, magnesium hydroxide, magnesium oxide, magaldrate, magnesium trisilicate, sodium bicarbonate, dibasic calcium phosphate, tribasic calcium phosphate, magnesium phosphate and pharmaceutically acceptable salts thereof as well as combinations of the various NSAIDs and various antacids. In more preferred embodiments ibuprofen or naproxen is used in combination with calcium carbonate, magnesium oxide and magnesium carbonate.

As embodied and broadly described herein, the invention further comprises a method for treating the symptoms of arthritic disorders and the symptoms of NSAID induced gastric acidity comprising administering a combination pharmaceutical composition to a patient comprising an analgesic, anti-inflammatory effective amount of an NSAID and a gastric acid neutralizing effective amount of one or more antacid.

Detailes Description of Preferred Embodiments of the Invention

Reference will now be made in detail to preferred embodiments of the invention, examples of which are illustrated in the following examples section.

To achieve the object of the invention of providing a pharmaceutical composition for treating the symptoms of arthritic disorders and the symptoms of NSAID induced gastric acidity an analgesic, anti-inflammatory effective amount of an NSAID is combined with a gastric acid neutralizing effective amount of one or more antacid(s). The symptoms of NSAID induced gastric acidity may include but are not limited to one or more of dyspepsia (e.g. indigestion heartburn and/or epigaetric pain), diarrhea, constipation, abdominal distress or pain, flatulence, peptic ulcer, and gastroenteritis. The combination composition of the present invention alleviates the above noted symptoms. For purposes of the present invention the term alleviate is defined as reducing, controlling, relieving or preventing the onset of the symptoms of NSAID induced gastric acidity.

The treatment of various arthritic diseases is related to the reversal of the disease process as well as the symptomatic relief of the pain that accompanies these disorders. This requires the use of NSAIDs which are well-known, clinically proven analgesic and anti-inflammatory agents. Most NSAIDs are weak organic acids that inhibit prostaglandin synthesis. (See Schoen, R.T. and Vender, R.J.: "Mechanisms of Nonsteroidal Anti-inflammatory Drug-induced Gastric Damage." Am. J. Med. 86:449-459, 1989.) The inhibition of prostaglandin synthesis leads to a breakdown of the gastric protective barrier provided by prostaglandin and mucosal damage due in large part to the generation of hydrochloric acid. Attempts to decrease the risks associated with anti-inflammatory treatment by using enteric coated formulations, suppositories, or pro-drugs have not solved this problem (see Ehsanullah, R.S.B., M.C. Page, G. Tildesley, J.R. Wood. "Prevention of gastroduodenal damage induced by nonsteroidal anti-inflammatory drugs: controlled trial of ranitidine." BMJ 297:1017-1021, 1988). Anti-ulcer drugs are often prescribed concomitantly. Since no single agent has been found to be capable of treating the symptoms of arthritic diseases without concomitant gastrointestinal injury, a combination composition as is described in this invention can fulfill such long felt need.

The composition of the present invention preferably comprises a combination of the following compositions or their pharmaceutically acceptable salts, an NSAID selected from the group consisting of: propionic acid derivatives including ibuprofen (the term ibuprofen is meant to include administration of both the racemic mixture of R- and S-enantiomers and the substantially pure S-enantiomer which is the analgesic active form of ibuprofen) from 200 to 800 mg per dose; naproxen from 200 to 500 mg per dose; fenoprofen from 200 to 600 mg per dose; flurbiprofen from 50 to 300 mg per dose; ketoprofen from 50 to 300 mg per dose; meclofenamate from 50 to 400 mg per dose; mefenamic acid from 250 to 500 mg per dose; piroxicam from 10 to 20 mg per dose; indomethacin from 25 to 200 mg per dose, sulindac from 150 to 400 mg per dose, tolmetin from 200 to 1200 mg per dose and mixtures thereof; in combination with an antacid including aluminum hydroxide from 300 to 600 mg per dose; basic aluminum carbonate from 500 to 1000 mg per dose; dihydroxyaluminum sodium carbonate from 300 to 600 mg per dose; dihydroxyaluminum aminoacetate from 100 to 2000 mg per dose; calcium carbonate from 600 to 2000 mg per dose; magnesium carbonate from 50 to 2000 mg per dose; magnesium hydroxide from 250 to 2000 mg per dose; magnesium oxide from 250 to 2000 mg per dose; magaldrate from 400 to 800 mg per dose; magnesium trisilicate from 500 to 4000 mg per dose; sodium bicarbonate from 300 to 4000 mg per dose and mixtures thereof.

The dosage ranges described above are preferred adult doses and may vary depending upon age and weight of the patient and/or the NSAID administered as would be known by those skilled in the pharmaceutical art. For example, the administrations of piroxicam within the recommended dosage range of 10 to 20 mg would require a smaller dose of antacid to provide a gastric acid neutralizing effect than would tolmetin in its preferred dosage range of 200 to 1200 mg. Further, if a combination of, for example mixtures of NSAIDs or antacids are used, the dosage for each component part of the mixture may be reduced.

To establish the efficacy of the composition of this invention in humans, patients suffering from the symptoms of arthritic diseases for whom NSAIDs are used and in whom the symptoms of NSAID induced gastric acidity occur can be administered the NSAID with and without the antacid, patients are asked to subjectively estimate onset of relief, duration of relief and time to maximum relief or to report the absence of the symptoms of gastric acidity. Appropriate statistical methods are used to show that NSAIDs/antacid combination medication are efficacious in preventing or reducing the symptoms of NSAID induced gastric acidity.

Efficacy studies relating to gastric mucosal damage induced by administration of NSAIDs in comparison to administration of NSAIDs/antacid combinations are carried out in animal models. The models include the determination of NSAID induced gastric mucosal damage in: an exteriorized rabbit colon direct contact gastrointestinal irritancy model and a five day oral gastrointestinal irritation study in cynomolgus Monkeys. Since appropriate animal models for the evaluation of the subjective relief af the symptoms of NSAID induced gastric irritation do not exist the animal studies described above are preliminarily conducted before the compositions of the invention are evaluated in human clinical trials.

Other ingredients both active and inactive can be added to the combination pharmaceutical compositions of of the invention. For example, flavoring compositions are Antidiarrheal, antiflatulent, antispasmodic and/or anticholinergic compositions may be added to the compositions of the invention to reduce and relieve gastrointestinal distress, which may be associated with acid indigestion. Examples of antidiarrheals include loperamide, attapulgite, bismuth subsalicylate, diphenoxylate HCl, polycarbophil, calcium polycarbophil and mixtures thereof. An example of an antiflatulent is simethicone. Examples of antispasmodics include phenobarbital dicyclomine HCl, belladonna alkaloids, and atropine.

Examples

The invention will now be illustrated by examples. The examples are not intended to be limiting of the scope of the present invention but read in conjunction with the detailed and general description above, provide further understanding of the present invention and an outline of a process for preparing the compositions of the invention. Example 1-10 disclose various formulations for preparing tablets or caplets in accordance with the invention. Various conventional techniques for preparing medicament tablets or caplets can be employed as would be known to those skilled in the art as is disclosed for example by Remington's Pharmaceutical Sciences, Mack Publishing Co., Chapter 90, "Oral Solid Dosage Forms", pp. 1603-1632 (1985). The disclosure of this reference is hereby incorporated herein by reference.

The following examples may be provided in filled gelatin capsule form or as tablets or caplets which include pharmaceutically acceptable tableting aide and excipiente as well as optional coloring and flavoring agents in addition to the active ingredients listed below for each of Examples 1-10 and are prepared in accordance with the teaching of "Remington's" as identified above.

Example 1:

    200 mg of naproxen
    250 mg of magnesium oxide
    50 mg of magnesium carbonate
    100 mg of calcium carbonate

Example 2:

    200 mg of naproxen
    400 mg of aluminum hydroxide
    400 mg of magnesium hydroxide

Example 3:

    200 mg of naproxen
    50 mg of magnesium carbonate

Example 4:

    200 mg of naproxen
    300 mg of aluminum hydroxide

Example 5:

    200 mg of naproxen
    400 mg of magnesium hydroxida

Example 6:

    25 mg of indomethacin
    250 mg of magnesium oride
    50 mg of magnesium carbonate
    100 mg of calcium carbonate

Example 7:

    200 mg of indomethacin
    400 mg of aluminum hydroxide
    400 mg of magnesium hydroxide

Example 8:

    200 mg of indomethacin
    50 mg of magnesium carbonate

Example 9:

    200 mg of indomethacin
    300 mg of aluminum hydroxide

Example 10:

    200 mg of indomethacin
    400 mg of magnesium hydroxide

Various other dosage forms can be applied herein such as a liquid emulsion/suspension or chewable tablet form employing the dosage actives provided above or other dosage amounts in accordance with the present invention. For example, the above-noted combination may be formulated in accordance with the teachings of U.S. Patent No. 4,806,359 for sustained release tablets or U.S. Patent No. 4,851,226 for chewable tablets. The entire disclosures of these references are hereby incorporated herein by reference.

Method of Treating Patients for the Symptoms of Arthritis Disease With NSAIDs and preventing NSAID Induced Gastric Acidity.

A patient exhibiting the symptoms or suffering from the symptoms of arthritic disease treated with an NSAID exhibiting the symptoms of NSAID induced gastric acidity may be treated by the oral administration of one tablet of the pharmaceutical composition in accordance with any of Examples 1-10.

The scope of the present invention is not limited by the description, examples and suggested uses herein and modifications can be made without departing from the spirit of the invention. For example, the pharmaceutical compositions of the invention may be provided in a bi-layer immediate/sustained release formulation for prolonged and/or nighttime treatment of the symptoms of arthritic disease treated with NSAIDs resulting in NSAID induced gastric acidity. Application of the compositions and methods of the present invention for medical and pharmaceutical uses can be accomplished by any clinical, medical and pharmaceutical methods and techniques as are presently or prospectively known to those skilled in the art. Thus it is intended that the presently claimed invention cover the modifications and variations of this invention provided that they come within the scope of the appended claims and their equivalents.

## Claims

1. A pharmaceutical analgesic composition, for alleviating the gastrointestinal symptoms induced by the administration of nonsteroidal anti-inflammatory drugs to humans suffering from pain, comprising a combination of an effective analgesic amount of a nonaspirin nonsteroidal anti-inflammatory drug and an effective gastric acid neutralizing amount of an antacid.

2. The pharmaceutical composition of Claim 1, wherein the nonsteroidal anti-inflammatory drug is a propionic acid derivative, a fenamic acid derivative, an oxicam, an indole acetic acid or a pharmaceutically acceptable salt thereof.

3. The pharmaceutical composition of Claim 1 or Claim 2, wherein:
the nonsteroidal anti-inflammatory drug is ibuprofen, flurbiprofen, fenoprofen, naproxen, ketoprofen, meclofenamate, mefenamic acid, piroxicam, indomethacin, sulindac, tolmetin or a pharmaceutically acceptable salt thereof, and is preferably substantially pure S-enantiomer of ibuprofen; and
the antacid is aluminum hydroxide, basic aluminum carbonate, dihydoxyaluminum sodium carbonate, dihydroxyaluminum aminoacetate, calcium carbonate, magnesium carbonate, magnesium hydroxide, magnesium oxide, magaldrate, magnesium trisilicate, sodium bicarbonate, dibasic calcium phosphate, tribasic calcium phosphate, magnesium phosphate, a mixture thereof, or a pharmaceutically acceptable salt thereof, and is preferably aluminium or magnesium hydroxide.

4. The pharmaceutical composition of any one of Claims 1 to 3, wherein
the nonsteroidal anti-inflammatory drug is ibuprofen from 200 to 400 mg per dose; naproxen from 200 to 500 mg per dose; fenoprofen from 200 to 500 mg per dose; flurbiprofen from 50 to 300 mg per dose; ketoprofen from 50 to 300 mg per dose; meclofenamate from 50 to 400 mg per dose; mefenamic acid from 250 to 500 mg per dose; piroxicam from 10 to 20 mg per dose; indomethacin from 25 to 200 mg per dose, sulindac from 150 to 400 mg per dose or tolmetin from 200 to 1200 mg per dose; and
the antacid is aluminium hydroxide from 300 to 500 mg per dose; basic aluminum carbonate from 500 to 1000 mg per dose; dihydroxyaluminum sodium carbonate from 300 to 500 mg per dose; dihydroxyaluminum aminoacetate from 100 to 2000 mg per dose; calcium carbonate from 600 to 2000 mg per dose; magnesium carbonate from 50 to 2000 mg per dose; magnesium hydroxide from 250 to 2000 mg per dose; magnesium oxide from 250 to 2000 mg per dose; magaldrate from 400 to 800 mg per dose; magnesium trisilicate from 500 to 4000 mg per dose; sodium bicarbonate from 300 to 4000 mg per dose or a mixture thereof.

5. The pharmaceutical composition of any one of Claims 1 to 4 comprising:
a combination of naxopren, magnesium oxide, magnesium carbonate and calcium carbonate;
a combination of naproxen, aluminium hydroxide and magnesium hydroxide; or
a combination of naproxen and magnesium carbonate.

6. The pharmaceutical composition of any one of Claims 1 to 5, which is in an oral tablet or caplet form.

7. The pharmaceutical composition of any one of Claims 1 to 5, which is in a chewable dosage form.

8. The pharmaceutical composition of any one of Claims 1 to 5, which is in a liquid dosage form.

9. The pharmaceutical composition of any one of Claims 1 to 5, which is in the form of a bi-layer tablet or caplet comprising one layer comprising an NSAID and a second layer comprising an antacid.

10. The pharmaceutical composition of any one of Claims 1 to 9, for use in the analgesic treatment with a nonsteroidal anti-inflammatory drug of a human patient suffering from pain and to alleviate one or more symptoms of nonsteroidal anti-inflammatory drug induced gastric acidity.

**European Patent Office**

# EUROPEAN SEARCH REPORT

Application Number

EP 91 30 6030

Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | PATENT ABSTRACTS OF JAPAN<br>vol. 12, no. 482 (C-553)(3329) 15 December 1988,<br>& JP-A-63 198620 (LION) 17 August 1988,<br>* the whole document * | 1-4,<br>6-10 | A61K45/06<br>A61K33/00 |
| X | PATENT ABSTRACTS OF JAPAN<br>vol. 13, no. 137 (C-582)(3485) 5 April 1989,<br>& JP-A-63 301817 (TAISHO PHARMACEUTICAL) 8<br>December 1988,<br>* the whole document * | 1-4,<br>6-10 | |
| X,P | EP-A-377906 (BAYER)<br>* page 3, lines 1 - 4 * | 1-4,<br>6-10 | |
| A | EP-A-259990 (MERCK & CO)<br>* page 2, lines 40 - 45 * | 1-4,<br>6-10 | |
| A<br>D | EP-A-317274 (MC NEIL CONSUMER PRODUCTS)<br>* the whole document *<br>& US-A-4851226 | 1-10 | |
| A<br>D | EP-A-290168 (MC NEILAB)<br>* the whole document *<br>& US-A-4806359 | 1-10 | TECHNICAL FIELDS SEARCHED (Int. Cl.5 )<br><br>A61K |
| X | MEDLINE DATABASE<br>U.S. National Library of Medicine,<br>Bethesda, MD.<br>CAILLE G. et al: "Effects of food and sucralfate<br>on the pharmacokinetics of naproxen and ketopro-<br>fen in humans."<br>Abstract Nb: 89285247 * the whole abstract *<br>& AM.J.MED., june 9, 1989, 86 (6A), p 38-44 | 5 | |

-/--

The present search report has been drawn up for all claims

| Place of search | Date of completion of the search | Examiner |
|---|---|---|
| BERLIN | 18 OCTOBER 1991 | AVEDIKIAN P. F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

**European Patent**

**Office**

# EUROPEAN SEARCH REPORT

Application Number

EP   91 30 6030

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | MEDLINE DATABASE<br>U.S. National Library of Medicine,<br>Bethesda, MD.<br>GUGLER R. et al: " Effects of antacids on the<br>clinical pharmacokinetics of drugs. An update."<br>Abstract Nb: 90214057 * the whole abstract *<br>& CLIN.PHARMACOKINET,.march 1990, 18 (3),p 210-9 | 5 | |
| | | | **TECHNICAL FIELDS SEARCHED (Int. Cl.5 )** |
| | | 5 | |

The present search report has been drawn up for all claims

| Place of search | Date of completion of the search | Examiner |
|---|---|---|
| BERLIN | 18 OCTOBER 1991 | AVEDIKIAN P.F. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)